# EUROPEAN PATENT SPECIFICATION

(11) **EP 0 417 764 B1**
(45) Date of publication and mention of the grant of the patent: **07.06.1995**
(21) Application number: 90117562.0
(22) Date of filing: 12.09.1990
(51) Int. Cl.: A61M 25/00, A61M 5/158

(54) **Needle assembly equipped with valve**
Einführnadel mit Ventil
Aiguille avec un guide et une soupape

(30) Priority: 13.09.1989 JP 237508/89
(43) Date of publication of application: 20.03.1991
(73) Proprietor: TERUMO KABUSHIKI KAISHA, Tokyo 151 (JP)
(72) Inventor: Deguchi, Hiromi, Fujinomiya-shi., Shizuoka-ken (JP); Yamamoto, Masanobu, Fujinomiya-shi., Shizuoka-ken (JP)
(74) Representative: Casalonga, Axel

(56) References cited:
- EP-A- 0 316 096
- DE-C- 3 809 127
- US-A- 4 424 833
- US-A- 4 430 081
- US-A- 4 610 665

## Description

The present invention relates to a needle assembly equipped with a valve, which is adapted to be used for infusing blood or liquid into a blood vessel.

Generally, the infusion of blood or liquid into a blood vessel is conducted by piercing a needle assembly comprising an inner needle member fitted integrally in the outer needle member into the blood vessel, withdrawing only the inner needle member from the blood vessel, and then connecting the hub of the outer needle member with a connector of, for example, a blood-infusion set (or a liquid-infusion set).

In this case, blood tends to leak out of the proximal opening of the hub of the outer needle member during the step of withdrawing the inner needle member, or during the step of connecting the hub of the outer needle member to the connector. In order to prevent such a leakage of blood, an outer needle member provided on its hub portion with a valve has been conventionally proposed.

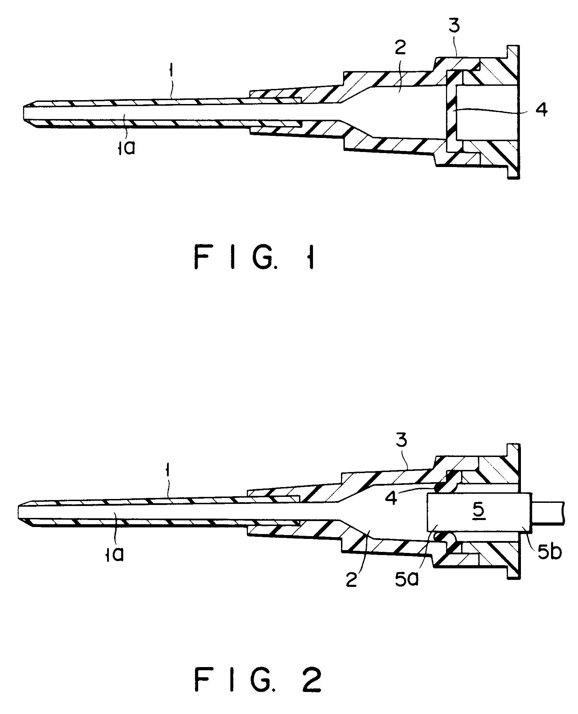

Fig. 1 shows an example of such an outer needle member equipped with a valve. This outer needle member comprises an outer needle 1, an outer needle hub 3 fixed to the proximal end of the outer needle 1 and having a passage 2 communicating with a longitudinal hollow portion 1a of the outer needle 1, and a film-like valve 4 mounted at a middle portion of the passage 2 of the outer needle hub 3.

When in use, an inner needle member (not shown) comprising an inner needle and an inner needle hub fixed, to the proximal end of the inner needle is fitted in the outer needle member thereby forming a needle assembly, which is then pierced into a blood vessel. Thereafter, the inner needle member is withdrawn from the outer needle member, leaving the outer needle member indwelled in the blood vessel. In this case, even if the inner needle member is withdrawn from the outer needle member, any leakage of blood can be effectively prevented due to the presence of the valve 4. However, when a connector of for example an infusion set is fitted in the outer needle hub 3,the connector is kept within the outer needle hub 3 as shown in Fig. 2. Namely, only the tip portion 5a of connector 5 is grasped by the valve 4 leaving proximal end portion 5b kept free to move due to the presence of the valve 4, thereby rendering the connector to be fixed in the outer needle hub 3 in a very unstable state as a whole. As a result, the connector 5 is merely weakly fitted in the outer hub 3 so that the connector is more likely to be accidentally detached if it is carelessly handled.

EP-A-0 316 096 discloses a hemostasis valve which includes a longitudinally extended housing having first and second opposing open ends, a cap means enclosing the first end and having an opening to permit insertion of a catheter into the longitudinally extended housing; and a one-piece seal means located within the central passage of the longitudinally extended housing. The seal means is provided with a sealing neck and sealing exit lips arranged so that the catheter may be readily inserted through the sealing neck and out the sealing exit lips. The second end of the valve housing is attached to a sheath which is inserted into the vasculature. By employing this hemostasis valve, it is possible to use different catheters which may vary in diameter.

DE-C-3809127 discloses a cannula for insertion of a catheter which comprises a end cap, a valve housing, and a seal closing the passage between the cap and the inside of the valve housing. The inner hollow portion within the valve housing is larger than the inner hollow portion within the cap. The valve is comprised of a flexible gasket and a spring. When a tubular member is inserted through the valve, a portion of the gasket is deformed and is urged against the outer surface of the tubular member by the action of the spring.

In both documents, the enlarged inner portions are such that the valve will not expand against the interior wall of these enlarged inner portions.

Accordingly, an object of the present invention is to provide a needle assembly equipped with a valve, which can be strongly and liquid-tightly connected to a connector of blood or liquid infusion set.

In order to solve the problems of the prior art as mentioned above, measures are taken according to the present invention to form an enlarged hollow portion at a portion of the hub of the outer needle member which is located inner side of the valve-mounting portion, the inner diameter of the enlarged hollow portion being larger than an inner diameter of a portion of the hub which is located outer side of the valve-mounting portion. This enlarged hollow portion presents a relief for the valve when a connector of a blood or liquid infusion set is connected to the outer needle hub.

Accordingly, the present invention provides a combination of a needle assembly and a rod-like member or tubular member, said needle assembly being equipped with a valve and comprising an outer needle member including an outer needle (or cannula and the like), an outer needle hub fixed to the proximal end of the outer needle and having an inner passage communicating with the outer needle, and a valve mounted to close the inner passage of the outer needle hub and being capable of allowing a rod-like member or a tubular member to pass therethrough, the outer needle hub including an enlarged hollow portion formed in a portion thereof which is located inner side of the valve-mounting portion and said rod-like member or tubular member having an outer diameter which is substantially the same as an inner diameter of a passage of a portion of the outer needle hub which is located outer side of the valve-mounting portion, characterized in that the inner diameter of the enlarged hollow portion is larger by twice a compressed thickness of the valve as it is pressed by the attachment of the rod-like member or the tubular member than the inner diameter of said portion of the outer needle hub which is located outer side of the valve-mounting portion and in which the rod-like member or the tubular member is fitted, the enlarged hollow portion forming a relief for receiving said deformed portion of the valve under compression when the rod-like member or the tubular member is inserted through the valve.

It is preferable that said portion of the inner wall surface of the outer needle hub which is located outer side of the valve-mounting portion is formed into a luer taper, thereby improving the fitting strengh and liquid-tightness of the rod-like member (such as a connector of a blood or liquid infusion set) or the tubular member relative to the outer hub.

When using the needle assembly as constructed above, the inner needle hub is fitted in the outer needle hub, and then pierced into a blood vessel. Then, the inner needle member is withdrawn from the blood vessel as well as from the outer needle member, and a connector of for example a blood or liquid infusion set is fitted in the outer needle hub. At this moment, the circumferential portion of the valve is bent inward by the insertion of the distal portion of the connector. However, this bent circumferential portion of the valve is allowed to escape into the enlarged hollow portion of the outer needle hub, so that the proximal end portion of the connector can be hermetically settled on the inner wall of the proximal portion of the outer needle hub. As a result, the connector can be fitted in the outer needle hub with a sufficient fitting strength.

This invention can be more fully understood from the following detailed description when taken in conjunction with the accompanying drawings, in which:
Fig. 1 is a sectional view showing an example of the outer needle member equipped with a valve according to the conventional needle assembly;
Fig. 2 is a partial sectional view of the outer needle member shown in Fig. 1, to which a connector of a blood or liquid infusion set is connected;
Fig. 3 is a partial sectional view of a needle assembly according to the present invention;
Fig. 4 is a sectional view of the needle assembly of Fig. 1, wherein the inner needle and inner needle hub are withdrawn therefrom;
Fig. 5 is a perspective view of a valve to be used in the present invention;
Fig. 6 is a side view of the valve shown in Fig. 5; and
Fig. 7 is a partial sectional view of the outer needle member shown in Fig. 4, to which a connector of a blood or liquid infusion set is connected.

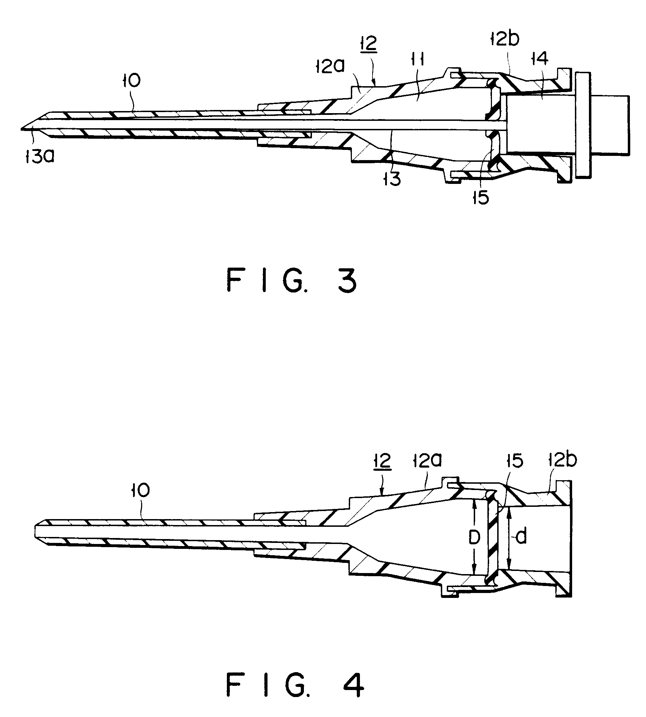

This invention will be further explained with reference with the example shown in Figs. 3 to 7.

Fig. 3 illustrates a partial sectional view of a needle assembly according to the present invention, wherein the needle assembly comprises an outer needle 10, an outer needle hub 12 fixed to the proximal end of the outer needle 10 and having an inner passage 11 communicating with the outer needle 10, an inner needle 13 which is adapted to be inserted into the outer needle 10 through the outer needle hub 12 to such an extent that only the tip portion 13a of the inner needle 13 projects outward through the tip of the outer needle 10, an inner needle hub 14 fixed to the proximal end of the inner needle 13 and adapted to be fitted in the outer needle hub 12, and a valve 15 mounted to the outer needle hub 12, closing the inner passage of the outer needle hub 12 and being capable of allowing a rod-like member (for example a connector of blood or liquid infusion set) or a tubular member to pass therethrough.

To be more specific, the outer needle hub 12 comprises a distal portion 12a and a proximal portion 12b, each connected together by being fitted to each other. The valve 15 is held between these connected portions of the distal portion 12a and the proximal portion 12b by the circumferential portion thereof. The inner diameter "D" of the distal portion 12a near the mounting portion of the valve 15 is enlarged as compared with the inner diameter "d" of the proximal portion 12b near the mounting portion of the valve 15 (see Fig. 4). Therefore, when a connector of a blood or liquid infusion set is fitted in the outer needle hub 12 as explained later, this enlarged portion (namely, "D") provides a relief or an escape space for the deformed portion of the valve 15. Accordingly, the inner diameter "D" is larger than the inner diameter "d" by twice the compressed thickness of the deformed portion of the valve 15.

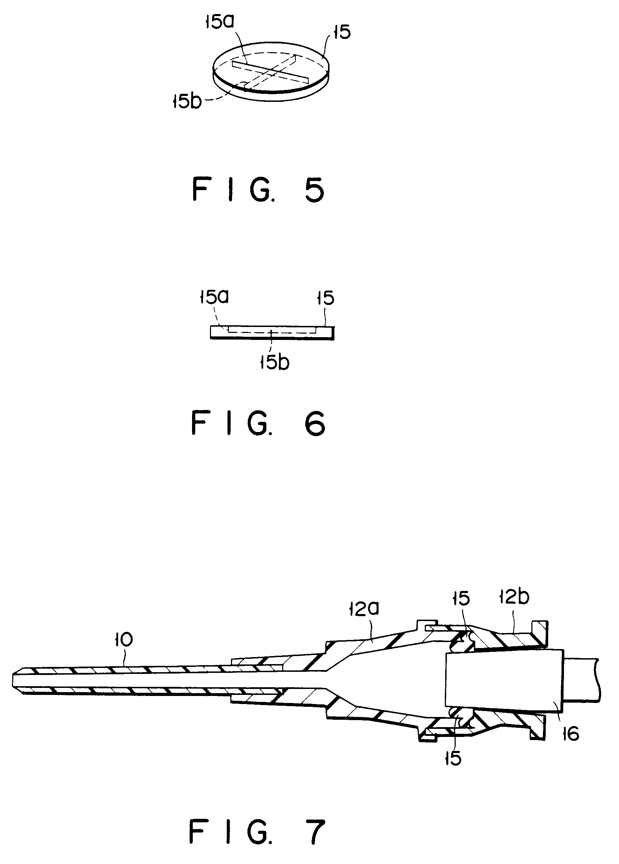

As for the valve 15, one shown in US-A- 4,610,665, such as shown in Figs. 5 and 6 can be employed. Namely, a valve formed of a resilient disc having a pair of linear cuts, i.e. a first cut 15a opening only to the top surface of the disc, and a second cut 15b opening only to the back surface of the disc, and crossed with each other at their centers can be employed. Of course, it is possible to employ any other type of film-like valve. The valve 15 is so constructed that when the inner needle 13 is withdrawn from the outer needle hub 12 as shown in Fig. 4, the valve 15 is spontaneously closed so as to prevent body liquid such as blood from leaking out of the outer needle 10.

It is desirable to make the inner wall of the proximal end portion 12b of the outer needle hub 12 into a luer tapered surface expanding toward the proximal end side of the outer needle hub 12 in view of increasing the fitting strength between the outer needle hub 12 and a connector as explained hereinafter.

Next, the operation of the needle assembly as constructed above will be explained below.

First, as shown in Fig. 3, the inner needle 13 is inserted into the outer needle 10 through the outer needle hub 12. When the inner needle hub 14 is sufficiently engaged to fit in the proximal end portion 12b of the outer needle hub 12, the tip portion 13a of the inner needle 13 is projected out of the tip portion of the outer needle 10, thereby rendering the needle assembly ready for smoothly piercing and penetrating into a blood vessel. Thereafter, the needle assembly is operated so that the tip portion of the outer needle 10 pierces and penetrates into, for example, a blood vessel. Subsequently, the inner needle 13 is withdrawn from the outer needle hub 12. At this moment, the valve 15 is spontaneously closed so as to prevent blood from leaking out of the outer needle hub 12. Then, a connector 16 of a blood or liquid infusion set (not shown) is fitted in the outer needle hub 12 as shown in Fig. 7 in such a manner that distal end portion of the connector 16 is inserted through the valve 15.

When the distal end portion of the connector 16 is inserted through the valve 15, the valve 15 is deformed as shown in Fig. 7, i.e. the deformed portion of the valve 15 is received under compression into the enlarged portion, having a diameter of "D", of the distal portion 12a of the hub 12. Therefore, the central outer wall portion of the connector 16 is hermetically contacted to the inner wall surface of proximal end portion 12b of the hub 12, thereby allowing the connector 16 to be strongly fitted in the proximal end portion 12b.

In this case, if the inner surface of the proximal end portion 12b of the hub 12 is of a luer tapered form, the fitting of the proximal end portion 12b to the connector 16 can be further improved.

This in turn improves the liquid-tightness of the connection between the hub 12 and the connector 16.

As explained above, the outer needle hub of the present invention is constructed in such a manner that the inner diameter of a portion of the outer needle hub which is located near the valve-mounting portion and at the outer needle side as viewed from the valve-mounting portion is enlarged by twice the compressed thickness of the deformed portion of the valve as compared with the inner diameter of a portion of the outer needle hub which is located near the valve-mounting portion and at the proximal end side as viewed from the valve-mounting portion. Therefore, even if a rod-like member such as a connector of an infusion set as described above or a tubular member is inserted through the valve, the deformed portion of the valve can be conveniently received under compression into this enlarged portion, thereby allowing the rod-like member or the tubular member to be closely contacted and secured to the inner surface of the proximal end of the outer needle hub. As a result, the fitting strength of the outer needle hub to a rod-like member or a tubular member can be increased, so that once the rod-like member or the tubular member is fitted in the outer needle hub, it would be difficult for the rod-like member or the tubular member to be detached from the outer needle hub by accident, and leakage of body liquid from the outer needle member can be prevented without fail.

## Claims

1. A combination of a needle assembly and a rod-like member or tubular member, said needle assembly being equipped with a valve (15), and comprising an outer needle member including an outer needle (10), an outer needle hub (12) fixed to the proximal end of the outer needle (10) and having an inner passage (11) communicating with the outer needle (10), and a valve (15) mounted to close the inner passage (11) of the outer needle hub (12) and being capable of allowing said rod-like member or tubular member to pass therethrough, the outer needle hub including an enlarged hollow portion formed in a portion thereof which is located inner side of the valve-mounting portion, and said rod-like member or tubular member having an outer diameter which is substantially the same as an inner diameter of a passage of a portion of the outer needle hub (12) which is located outer side of the valve mounting portion, characterized in that the inner diameter of the enlarged hollow portion is larger by twice a compressed thickness of a deformed portion of the valve (15) as it is pressed by the attachement of the rod-like member or the tubular member than the inner diameter of said portion of the outer needle hub (12) which is located outer side of the valve-mounting portion and in which the rod-like member or the tubular member is fitted, the enlarged hollow portion forming a relief for receiving said deformed portion of the valve (15) under compression when the rod-like member (16) or the tubular member is inserted through the valve (15).

2. The combination according to claim 1, characterized in that said portion of the outer needle hub (12) which is located outer side of the valve-mounting portion has a tapered inner wall surface.

3. The combination according to claim 1, characterized in that said outer needle hub (12) comprises a distal portion (12a) and a proximal portion (12b), connected to each other, and the valve (15) is held between these connected portions of the distal portion (12a) and the proximal portion (12b) by the circumferential portion thereof.

4. The combination according to claim 1, characterized in that said valve (15) is formed of a disc having a pair of linear cuts (15a, 15b), i.e. a first cut (15a) opening only to the top surface of the disc, and a second cut (15b) opening only to the back surface of the disc, and crossed with each other at their centers.

5. The combination according to claim 1, characterized in that said rod-like member (16) is of a cylindrical form.

## Patentansprüche

1. Kombination einer Nadelanordnung und eines stabförmigen Elements oder rohrförmigen Elements, wobei die Nadelanordnung mit einem Ventil (15) versehen ist, und umfassend ein äußeres Nadelelement, enthaltend eine Außennadel (10), eine Außennadelbuchse (12), die am proximalen Ende der Außennadel (10) befestigt ist und eine mit der Außennadel (10) in Verbindung stehende Innendurchführung (11) aufweist, und ein Ventil (15), das zum Verschließen der Innnendurchführung (11) der Außennadelbuchse (12) angebracht ist und in der Lage ist zu gestatten, daß das stabförmige Element oder rohrförmige Element hindurchtritt, wobei die Außennadelbuchse einen erweiterten hohlen Abschnitt aufweist, der in einem Abschnitt von ihr gebildet ist, der sich innenseitig des Ventilanbringabschnittes befindet, und wobei das stabförmige Element oder das rohrförmige Element einen Außendurchmesser aufweist, der im wesentlichen derselbe wie derjenige eines Innendurchmessers einer Durchführung eines Abschnittes der Außennadelbuchse (12) ist, der sich außenseitig des Ventilanbringabschnittes befindet, dadurch **gekennzeichnet,** daß der Innendurchmesser des erweiterten hohlen Abschnittes um das Zweifache einer komprimierten Dicke eines deformierten Abschnittes des Ventils (15), wenn er durch die Anbringung des stabförmigen Elementes oder des rohrförmigen Elementes druckbeaufschlagt wird, größer als der Innendurchmesser des Abschnittes der Außennadelbuchse (12) ist, der sich außenseitig des Ventilanbringabschnittes befindet, in dem das stabförmige Element oder das rohrförmige Element befestigt ist, wobei der erweiterte hohle Abschnitt ein Relief zur Aufnahme des deformierten Abschnittes des Ventils (15) unter Kompression bildet, wenn das stabförmige Element (16) oder das rohrförmige Element durch das Ventil (15) eingeführt wird.

2. Kombination nach Anspruch 1, dadurch **gekennzeichnet,** daß der Abschnitt der Außennadelbuchse (12), der sich außenseitig des Ventilanbringabschnittes befindet, eine verjüngte Innenwandfläche aufweist.

3. Kombination nach Anspruch 1, dadurch **gekennzeichnet,** daß die Außennadelbuchse (12) einen distalen Abschnitt (12a) und einen proximalen Abschnitt (12b) umfaßt, die miteinander verbunden sind, und das Ventil (15) zwischen diesen verbundenen Abschnitten des distalen Abschnittes (12a) und des proximalen Abschnittes (12b) durch dessen Umfangsabschnitt gehalten ist.

4. Kombination nach Anspruch 1, dadurch **gekennzeichnet,** daß das Ventil (15) aus einer Scheibe mit einem Paar linearer Einschnitte (15a, 15b) gebildet ist, d.h. einem ersten Einschnitt (15a) der sich lediglich zur Oberseite der Scheibe öffnet, und einem zweiten Einschnitt (15b) der sich lediglich zur Rückseite der Scheibe öffnet, und die an ihren Zentren miteinander gekreuzt sind.

5. Kombination nach Anspruch 1, dadurch **gekennzeichnet,** daß das stabförmige Element (16) eine zylindrische Form aufweist.

## Revendications

1. Association d'un ensemble d'aiguilles et d'un organe en forme de tige ou organe tubulaire, ledit ensemble d'aiguilles étant muni d'une soupape (15) et comprenant un élément formant aiguille extérieure qui comprend une aiguille extérieure (10), un manchon (12) d'aiguille extérieure fixé à l'extrémité proximale de l'aiguille extérieure (10) et comportant un passage intérieur (11) qui communique avec l'aiguille extérieure (10), et une soupape (15) montée pour fermer le passage intérieur (11) du manchon (12) d'aiguille extérieure et capable de permettre le passage dudit organe en forme de tige ou dudit organe tubulaire, le manchon d'aiguille extérieure comprenant une partie creuse agrandie formée dans sa partie qui est placée sur le côté intérieur de la partie de montage de la soupape et ledit organe en forme de tige ou organe tubulaire ayant un diamètre extérieur qui est sensiblement le même que le diamètre intérieur du passage de la partie du manchon (12) d'aiguille extérieure qui est située sur le côté extérieur de la partie de montage de soupape, caractérisée en ce que le diamètre intérieur de la partie creuse agrandie est plus grand de deux fois l'épaisseur comprimée de la partie déformée de la soupape (15) lorsqu'elle est comprimée par la fixation de l'organe en forme de tige ou de l'organe tubulaire que le diamètre intérieur de ladite partie du manchon (12) d'aiguille extérieure qui est placée sur le côté extérieur de la partie de montage de soupape et dans lequel l'organe en forme de tige ou l'organe tubulaire est emboîté, la partie creuse agrandie formant une partie en relief destinée à recevoir ladite partie déformée de la soupape (15) sous compression quand l'organe en forme de tige (16) ou l'organe tubulaire est introduit à travers la soupape (15).

2. Association selon la revendication 1, caractérisée en ce que ladite partie du manchon (12) d'aiguille extérieure qui est située sur le côté extérieur de la partie de montage de soupape a une surface de paroi intérieure biseautée.

3. Association selon la revendication 1, caractérisée en ce que ledit manchon (12) d'aiguille extérieure comprend une partie distale (12a) et une partie proximale (12b) raccordées l'une à l'autre et la soupape (15) est maintenue entre ces parties raccordées de la partie distale (12a) et de la partie proximale (12b) par sa partie circonférentielle.

4. Association selon la revendication 1, caractérisée en ce que ladite soupape (15) est faite d'un disque comportant une paire d'encoches rectilignes (15a, 15b), à savoir une première encoche (15a) qui ne s'ouvre que sur la surface de dessus du disque et une seconde encoche (15b) qui ne s'ouvre que sur la surface arrière du disque, encoches qui se croisent au niveau de leur centre.

5. Association selon la revendication 1, caractérisée en ce que ledit organe en forme de tige (16) a une forme cylindrique.
